# EUROPEAN PATENT APPLICATION

(11) **EP 4 066 889 A1**
(43) Date of publication of application: **05.10.2022**
(21) Application number: 21165752.3
(22) Date of filing: 30.03.2021
(51) Int. Cl.: A61N 5/10, A61B 6/00, B29C 64/165, G01T 1/10, B33Y 10/00, B33Y 70/10, B33Y 80/00

(54) **3D PRINTING MATERIAL FOR DOSE MEASUREMENT PHANTOMS**

(71) Applicant: AGFA NV, 2640 Mortsel (BE)
(72) Inventor: LEBLANS, Paul, 2640 Mortsel (BE); VERMEERSCH, Joan, 2640 Mortsel (BE)
(74) Representative: Verbrugghe, Anne Marie L.

(57) **Abstract**

The invention relates to a 3D printing material that allows the production of anthropomorphic phantoms for verification of a radiotherapy treatment session for a patient. The 3D printing material comprises luminescent particles that produce a luminescent signal upon stimulation or excitation by a light source, a signal that correlates with the absorbed dose. The 3D printing material is used with standard additive 3D printing techniques in order to print the anthropomorphic phantom. It also relates to a method to acquire the dose distribution data in the volume of this adapted anthropomorphic phantom.

## Description

### Technical Field

The present invention relates to 3D printing materials that are used in the manufacturing of dose measurement phantoms and dose verification phantoms used in the field of radiotherapy. It relates to the manufacture and use of such dose measurement phantoms, and more specifically to the production of such phantoms by means of 3D printing.

A 3D printing material is proposed that allows the production of anthropomorphic phantoms for verification of a radiotherapy treatment session for a patient. The 3D printing material comprises luminescent particles that produce a luminescent signal upon stimulation or excitation by a light source, a signal that correlates with the absorbed dose. It also relates to a method to acquire the dose distribution data in the volume of this adapted anthropomorphic phantom. The method measures the deposited dose of the radiotherapy treatment session by means of stimulating a layer or plane of luminescent particles in the phantom by a scanning laser beam, allowing the emission light to be captured by a visible light camera that is setup perpendicular to the stimulation plane.

### Background of the invention

Radiation delivering techniques in radiotherapy have become more and more sophisticated with the introduction of intensity modulated radiation therapy (IMRT), volumetric modulated arc therapy (VMAT) and especially proton therapy. While traditional radiotherapy delivery is performed by high energy X-ray beams of which the dose deposition characteristics are relatively well known, the application of electron beams and proton beams often require more quality assurance before application on a real patient can be considered.

As a result, verification of the radiation treatment plan has become increasingly important. The verification method of choice is high-resolution three-dimensional dosimetry. However, adequate tools which enable to directly measure the dose distributions in three-dimensional (3D) space are not commonly available. One such three-dimensional dose measurement device is the polymer-based dosimeter, which changes the material property in response to radiation.

Such three-dimensional dose measurement devices are currently available in gel state as a polymer gel dosimeter (PGD) or as a ferrous gel dosimeter (FGD) and are known as radiochromic gels. Other dose measurement devices may appear in a solid state as a solid plastic dosimeter (SPD).

The currently known three-dimensional dosimeters however have different short-comings, and are e.g., not re-usable, involve complicated readout, have physical properties (such as refractive index changes) leading to artefacts, are sensitive to oxidation, are unstable and/or have a dose-rate dependent response.

The capacity to record acquired dose can be based on different physicochemical phenomena. The current standard methods of the dose quantification with such gel or solid state devices are magnetic resonance imaging, optical computed tomography, and X-ray computed tomography. In particular, magnetic resonance imaging is well established as a method for obtaining clinically relevant dosimetric data by PGD and FGD. Despite the technical feasibility of performing 3D dosimetry by PGD, FGD or SPD, these tools are not broadly used for clinical applications. The lack of a broad acceptance of these techniques in radiation oncology applications stems from the inherent inaccuracy of the tools, and the high costs involved in everyday usage.

For these reasons 2D dosimetry still is the default technique for patient-specific quality assurance in radiotherapy. Currently two technologies are used for this purpose: radiochromic film and 2D digital dosimetry.

Radiochromic film is based on colour changes by a polymerization process that is induced by exposure to radiation. After the film is exposed in a phantom a 2D scan in an optical film scanner measures the colour change as a function of position, which is then translated to a high-resolution 2D dose profile. Radiochromic film dosimetry has a number of disadvantages. The polymerization reaction requires 48h to stabilize. Hence, a waiting time of 12 to 24 h is required after exposure. The response of the film is nonlinear and the film saturates at doses that are relatively low for radiation therapy. Radiochromic film dosimetry is very labor intensive. It is also very sensitive to processing (handling, scanning, ...). As a consequence, radiochromic film dosimetry can only be performed by thoroughly trained personnel. Finally, the film is single use and can, therefore, not be calibrated. 2D array detectors are 2D digital dose detectors, based on 2D arrays of diodes or on 2D arrays of ionization chambers. 2D diode arrays provide an on-line result, which can be compared readily to the calculated dose of the treatment plan. However, also 2D diode arrays have a number of drawbacks. The most important drawbacks are that the devices are expensive (30.000 to 100.000 €) and the low resolution, since the pixel pitch is of the order of 5 to 10 mm.

In medical imaging a CR system detects the dose distribution in an X-ray beam that has passed through a patient to create an image. Since a CR system detects a 2D radiation profile, it is, in principle, also suitable for 2D dosimetry. It has been demonstrated that a CR system with an adapted storage phosphor plate can be a suitable 2D dosimeter for radiation therapy quality assurance with a very high dynamic range. It has the potential to combine the advantages of radiochromic film and diode arrays by offering high-resolution in combination with nearly online work-flow and user friendliness. Since the interaction of the phosphor plate with γ- and X-ray radiation can be made body-equivalent a CR system could also be fit for 2.5D dosimetry, by placing CR plates at different levels in a radiation phantom.

However, this approach can only lead to 2.5D dosimetry, meaning that the dose profile has high spatial resolution information in the plane of the CR plates but a low resolution in the direction perpendicular to the CR plate. The resolution in the direction perpendicular to the CR plate will be determined by the distance between the active layers of the phosphor plates and this distance can be of the order of millimeters at best.

Another disadvantage of the above approach is that storage phosphor plates can only be used in non-patient specific phantoms. As a consequence, dose profiles have to be calculated for the patient-specific treatment of standard phantoms in which the phosphor plates are inserted. Therefore, the conditions of dosimetry are not entirely realistic.

The current invention allows to address both disadvantages of CR dosimetry.

In the prior art, a dosimetry phantom is disclosed in EP3264137 A1 that comprises a matrix and optically stimulated luminescence particles, this phantom being at least 10 cubic millimeters in size. The luminescence particles are within one micrometer to hundreds of micrometers in size.

### Summary of invention

The present invention provides a 3D printing material for printing a dose measurement phantom, comprising a base material matrix, said base material being tissue-equivalent and transparent for the wavelength of a stimulation light beam and for the wavelength of emission of the incorporated luminescent particles, and particles comprising luminescent material evenly embedded in said matrix, and characterized in that said luminescent particles are smaller than the wavelength of the stimulation light.

It is an object of this invention to provide such a 3D printing material to allow the production of an object or a phantom that can be subjected to radiation and which then can be subjected to examination revealing the dose distribution. This may for example be relevant for verifying (before actually subjecting a patient to the radiation) that an intended dose distribution of radiation does in fact correspond to the area requiring treatment, while leaving healthy tissue as unaffected as possible. Such a technique may also serve as a verification to establish whether the intended dose distribution actually corresponds to the predicted dose distribution of the radiotherapy treatment device.

An advantage to provide the material as a 3D printer material is that it can be used to produce an object of virtually any shape, and that such an object would exhibit the same dose accumulation characteristics as human tissue. Radiological water equivalence is an important quality of solid phantom materials proposed to be used for dosimetric applications that rely on the attenuation and scattering characteristics of water. ICRU Report 44 provides quantification of phantom water-equivalence, describing the phantom as water equivalent if it does not introduce uncertainties greater than 1% in the calculation of absorbed dose.

Evaluation of the water equivalence of a material over the range of therapeutic energies used in radiation oncology requires consideration of the interrelated dependence of radiation interaction cross section, energy and atomic composition. At kilo voltage X-ray energies, where the dominant photon interaction is via the photoelectric effect, a small concentration of high atomic number (Z) material can easily alter the absorption properties of a medium due to the Z³ dependence of the mass attenuation coefficient. At megavoltage energies, where Compton scattering is the dominant interaction, matching the relative electron density (RED) of the material precedes importance over atomic composition to achieve water equivalent attenuation and scattering characteristics. For megavoltage electron energies, equivalence of electron density and atomic composition are required in order to emulate the stopping power and scattering power of water.

Most of the proposed polymers are meeting these criteria in practice, such that standard 3D printing materials be used for the purpose of producing a radiotherapy dosimetry phantom.

The cost of production of a 3D printed object is relatively low as most of the mass it consists of is represented by a relatively inexpensive matrix material. The production will only consume more time for more complex structures to be printed, because the current 3D printing techniques rely on layering subsequent layers of material upon each other. Each layer has to harden or dry before a next one can be applied when using the current additive manufacturing technologies.

The material used to create the 3D phantom of this invention is a transparent 3D printing material containing luminescent particles. These luminescent particles may be optically stimulated luminescent (OSL) particles or may be radio-photo luminescent (RPL) particles.

Optically stimulated luminescence (OSL) is the phenomenon where electrons and holes are trapped between the valence and conduction bands in the crystalline structure of certain minerals when subjected to radiation. Under stimulation of light, the electrons may free themselves from the trap and recombine with the hole through a tunneling process or via the conduction band. This recombination produces energy that is transferred to a luminescent center in the neighbourhood. This center is excited and emits luminescence upon de-excitation.

Optically luminescent particles may be for instance storage phosphor particles. In many applications, crystalline phosphors are used which require the presence of defects in the crystal lattice structure. To achieve defects, activators (also called dopants) are added to the phosphor at the time it is prepared. For example, BaFBr is typically doped with Eu₂0₃ or EuBr₂ resulting in BaFBr:Eu. The principal emission band appears around 400nm, whereas the absorption peak for stimulation appears around 550nm.

Radio-photo luminescence (RPL) is the property of certain substances to form fluorescence centers when irradiated with ionizing radiation which emit light or NIR radiation when excited with light with a shorter wavelength. In the case of suitable material the emitted light intensity is proportional to the number of luminous centers formed by the ionizing radiation and thus to the incident dose.

The main difference in use properties between both luminescence types (OSL and RPL) is that the excitation of the radio-photo luminescent material does not cause the fluorescence centers to disappear: with radio-photo luminescence the stored image caused by the luminescence centers remains present in the material, and therefore can be read-out through the excitation multiple times. The situation is different with optically stimulated luminescence (OSL) where the image is partly erased when stimulated with the stimulation light. It is common practice to completely erase the material after read-out in order to re-use the OSL material for a new cycle of exposure to radiation and read-out.

The 3D printing material that will constitute the base material matrix has to be a material that is transparent for visible light to allow the stimulation light and the luminescent light to pass through it without losing intensity. The readout techniques that optically stimulate the luminescent particles make use of a very intense visible light source, such as a laser. The stimulation light should be able to pass through every thickness of the base material matrix, and should still be able to stimulate the luminescent particles at the different depths in the phantom.

The visible light transparent 3D printing material comprises a base material matrix that embeds the luminescent particles. The base material matrix provides the physical characteristics such as the strength, rigidity, weight, etc... of the final artifact, and provides optical access to the embedded luminescent particles.

The different available 3D printing technologies will determine which base materials will be used. For instance, Fused Deposition Modelling (FDM) provides the widest range of materials to choose from thanks to the diversity of transparent filament types. The most common materials applied with this technique are for instance polyactic acid (PLA) filaments. An alternative is acrylonitrile butadiene styrene (ABS) which is in itself translucent, but can be made more transparent by blending it with an additive. Further can polymethylmethacrylate (PMMA), polycarbonate (PC) and polyethylene terephthalate glycol (PETG) be used to obtain a transparent result.

The luminescent particles are homogeneously distributed in the base material by mechanically mixing the particles with the base material. The homogeneous blending of the luminescent particles ensures that when the material is exposed to a certain radiation dose, the dose response signal will be homogeneous at any location within the phantom.

It is an advantageous effect of the invention that the stimulation light is optimally used to stimulate the luminescent particles. This effect can be directly derived from the transparency of the base material.

Another advantageous effect of the invention is that the stimulation or excitation light will not interact with the luminescent particles such that it causes scattering light in different directions across the object or phantom. The light scattering within the phantom will be negligible such that the measured signal of the luminescent light will not be influenced by scattering effects. The effects of light scattering can be prevented by ensuring that the particle sizes of the luminescent particles are smaller than the wavelength of the stimulating light.

In practice, the stimulating light is produced by a laser such as a diode laser, a He-Ne laser or a frequency-doubled Nd:YAG laser. The light of the stimulating laser may be for instance 470nm, which requires that the luminescent particles should be smaller than 470nm.

The stimulation wavelength range for OSL powder is 400-1700 nm and the luminescence wavelengths are 270-1020 nm. The emission wavelength is shorter than the stimulation wavelength. For example, a Risö reader employing a UV-340 optical filter obtains a main transmission between 250-400 nm, and uses blue light emitting diodes operating at 470 nm for stimulation. A typical CR-reader device uses a laser diode that operates at 660 nm for stimulation and produces emission light by the phosphors around 400-450 nm.

After exposure of the phantom to radiation, the 3D dose distribution can be obtained by stimulating or exciting the luminescent particles selectively. A good approach to obtain the dose distribution of the luminescent particles in a single plane would be to stimulate all luminescent particles in this single plane, and then read out the produced luminescent 2D signal in this plane by a camera. The camera should then be positioned orthogonally to the stimulated plane in order to capture the response signal from all particles in the stimulated plane. The obtained 2D image as such represents the dose distribution within the plane where the stimulation light was applied.

The stimulation can be achieved by applying a sheet of light that may be obtained by scanning a laser beam linearly through the phantom. The illumination of a single sheet through the phantom may be achieved by performing a linear scanning movement of a single beam plane through the phantom, or may for instance be achieved by illuminating a single plane through the phantom by applying a light source that is casted through a linear slit such as to obtain a planar illumination of a plane through the phantom.

In order to read out the dose distributions for the other parallel planes in the phantom, the plane of the stimulation light should be shifted parallel to the previous plane. The dose distributions for the different parallel planes can thus be obtained and reconstructed into a 3D dose distribution. The result being a 3D dose distribution model for the scanned object or phantom in question.

The invention further provides a patient-specific 3D phantom of a body part of the patient that has to be treated by radiotherapy, and is printed with a 3D printer. The 3D phantom is produced through a 3D printing process and is has the identical morphology of the patient, which is obtained from a 3D scan.

Another advantage of the invention is that the phantom is re-usable when using OSL particles since electron hole pairs in the OSL material can be repeatedly excited by ionizing radiation and recombined by optical stimulation (i.e., OSL dosimeters can be reset by, e.g., high temperature and/or an optical bleaching procedure).

A phantom has to be understood as a mechanical model of a patient or object, such as an anthropomorphic model, that can be used to measure an accumulated dose caused by a radiotherapy treatment setup. The phantom may change properties and may luminescence (such as optically stimulated luminescent light) upon irradiation by a radiotherapy treatment beam.

A matrix has to be understood as a material in which something is enclosed or embedded. A matrix material is the material that may be solid, homogeneous, monolithic and/or polymeric, and that for the purpose of 3D printing may be thermoplastic. A thermoplastic material is a material that becomes pliable or moldable at a certain elevated temperature and solidifies upon cooling, such as is the case in a typically 3D printing process where a print head heats the thermoplastic material to deposit it while it is moldable.

The meaning of 'particles' has to be understood in the scope of this invention as solid structures which have relatively small volumes and dimensions when compared to the volume and dimensions of the matrix material. The particles in this invention have dimensions smaller than the wavelength of the stimulating light that triggers the optical luminescence in the material of the particles.

In the context of this invention, the particles are embedded in the matrix, which means that the particles are enclosed closely by the matrix material such that these embedded particles become an integral part of the embedding structure; namely the matrix material.

### Description of embodiments

In the following detailed description, reference is made in sufficient detail to the above referenced drawings, allowing those skilled in the art to practice the embodiments explained below.

The 3D printing material of the invention comprises a combination of a base material matrix mixed with OSL particles. The base material matrix or matrix makes up the largest volume of the material and provides the mechanical material characteristics such as density, strength and alike. The OSL particles provide the dosimetric capabilities of the material and are only present in a minimal concentration.

Geometrically accurate models can be designed using medical images acquired using MRI or CT and inexpensively fabricated using polymers including polyeurethane, polylactic acid and epoxy resins. Materials and densities can be varied to produce radiographic contrast or tissue-equivalence.

The broad adoption of a rapid prototyping technique for the production of patient specific phantoms for use in radiotherapy dosimetry requires that the resulting phantoms are shown to accurately replicate the desired patient geometries, be sufficiently tissue-equivalent to provide usable measurements and be insusceptible to the effects of radiation.

For that purpose, acrylonitrile butadiene styrene (ABS) is a suitable phantom material that meets the requirements to serve as a possible matrix material in this invention, and which can be produced quickly and inexpensively via 3D printing. Moreover, ABS is transparent for the stimulation light and emission light of the OSL particles.

ABS has a density of 1.05 g/cm3. In order to achieve tissue-equivalence, the polymer may be printed in a mesh pattern, with air filling the gaps between solid strands of ABS, to produce some lower density materials. The air gaps may have an influence on the light scattering by the material, and therefore have to be kept as small as possible. Different ABS infill densities exhibit densities and attenuation coefficients that fall into the range of values identified in commercially available tissue-equivalent materials. The results for a 90% ABS infill density material lie between the values for the water-equivalent and liver-equivalent inserts from the tissue characterization phantom, potentially providing a useful approximation of tumor, muscle or other soft tissue.

The results for the 30% and 50% ABS infill density materials are similar to the values for lung-equivalent phantom inserts, making them suitable for use in the construction of lung-equivalent phantoms. The use of a meshed printing technique to produce low-density samples results in meshes that are very fine and foam-like.

The OSL particles used in the 3D printing material of this invention are preferably phosphors having a low effective atomic number and emitting in a suitable spectral range. The very common storage phosphor is BaFBr:Eu²⁺ and is widely applied in computed radiography storage phosphor plates. Its effective atomic number is however not ideal for an application in high energy dosimetry as they display a high energy dependence. This energy dependence results in relatively higher signals for exposures at low X-ray energies (kV-range) which would lead to an overestimation of the absorbed dose.

Therefore, other OSL particles are being investigated and more specifically the ones which exhibit a low effective atomic number and are emitting in a suitable visible light range for detection. Some of these alternative phosphors emit at slightly too short wavelength in order to be classified as ideal. For this category, the order of preference is determined by the effective atomic number. The following materials may be considered by order of preference: Na₂LiPO₄:Eu, NaLi₂PO₄:Eu, MgB₄O₇:Ce,Li, MgO:Fe, SrO-B₂O₃:Sn, AlN, Na₈Al₆Si₆O₂₄(Cl,S)₂, Li₃PO₄.Cu, KMgF3:Eu and LiCaAIF6:Eu,Y.

As the particle size of the luminescent particles have to be kept below the size of the wavelength of the stimulation light, which is typically below 470nm for our application due to the wavelength emitted by the stimulation laser, the particle size of the luminescent particles has to be controlled during production. The small particle sizes may be obtained by milling or grinding, by selecting the precipitation conditions for the precipitation of the phosphor from a solution, by additive controlled crystallization, or by sedimentation classification of the yielded powder.

The 3D printing material may be supplied as a flexible filament that may be provided on a roll.

The anthropomorphic phantom of this invention is 3D printed with the 3D printing material as described above. The CT or MRI scans of the region of interest of the patient provide the data for printing the 3D phantom and provide information about the shape of the different structures and their (electron) densities. A software package then calculates the instructions for the 3D printer in order for it to print the phantom layer by layer. In this process, the tissue densities measured in the scans may be taken into account such that areas of different density may be executed at different infill densities such as to reflect different tissue densities.

## Claims

1. A 3D printing material for printing a dose measurement phantom, comprising:
- a base material matrix consisting of a material that is transparent for the wavelength of a stimulation light beam and for the wavelength of the luminescent light caused by emission of incorporated luminescent particles,
- luminescent particles that are evenly embedded in said base material matrix, **characterized in that**,
said luminescent particles are smaller than the wavelength of the stimulation light that is required for stimulation of said luminescent particles.

2. A 3D printing material according to Claim 1, wherein said luminescent particles are optically stimulated luminescent particles.

3. A 3D printing material according to Claim 1, wherein said luminescent particles are radio-photo luminescent particles.

4. A 3D printing material according to any of the previous claims, wherein said luminescent particles being smaller than 470nm.

5. A 3D printing material according to any of the previous claims, wherein said base material matrix is a resin, a plastic or powder.

6. A 3D printing material according to any of the previous claims, wherein said base material matrix is a thermoplastic material.

7. A 3D printing material according to any of the previous claims, wherein said base material matrix combined with said particles is tissue-equivalent as far as X-rays are concerned.

8. A 3D printing material according to any of the previous claims, wherein said base material matrix is tissue-equivalent.

9. A phantom for 3D dosimetry, comprising 3D printed material according to any of the previous claims.

10. A phantom for 3D dosimetry according to Claim 6, said phantom being an anthropomorphic phantom.

11. Method for producing an anthropomorphic phantom of a patient suitable for dosimetry verification, comprising the steps of:
- obtaining a computerized tomography scan dataset from said patient,
- 3D printing an anthropomorphic phantom from said 3D printing material according to Claim 1, developed from said computer tomography.

12. Method to measure a dose distribution within a 3D printed object from a 3D material according to Claim 1, comprising the steps of
- exposing said anthropomorphic phantom to at least one radiotherapy treatment beam,
- exposing said anthropomorphic phantom to a planar scanning laser beam such that said OSL particles in said scanned plane are stimulated,
- determining the dose distribution in said scanned plane of said phantom by capturing an 2D image of said stimulated OSL particles in said scanned plane by an image camera that is positioned perpendicular/orthogonally to said scanning plane, and wherein an intensity of a measured signal at a location in said 2D image corresponds to a deposited dose at said location said 2D image,
- progressing through the entire phantom by shifting said scan plane line-by-line through said phantom.
